# EUROPEAN PATENT APPLICATION

(11) **EP 3 476 431 A1**
(43) Date of publication of application: **01.05.2019**
(21) Application number: 17382729.6
(22) Date of filing: 31.10.2017
(51) Int. Cl.: A61N 1/36, A61N 1/04, C12N 13/00, A61N 1/32, A61M 21/00

(54) **EQUIPMENT AND METHOD OF NEUROSTIMULATION**

(71) Applicant: Aikoli NTS, S.L.U., 20018 Donostia - San Sebastian (Gipuzcoa) (ES)
(72) Inventor: ARMENTA MÚGICA, Yolanda, 20005 Donostia - San Sebastian (ES)
(74) Representative: Codoñer Molina, Vicente

(57) **Abstract**

The invention describes a portable electronic apparatus consisting of 4 modules (HL, FL, HR and FR), and a neurostimulation method that uses two-phase direct current electrical signals with a very low intensity (micro-currents), with an encoded sequence of pulses, to generate and transmit electrical signals to the ribosomes of the cells of living beings, simulating the role of mDNA (Messenger DNA), to create essential amino acids and polypeptide chains. Modules connect to each other via a wireless local network. The two-phase electrical signals form encoded packets of 6-bit squared pulses spaced depending on the neurostimulation method. The encoding of the electrical signals uses the 64 hexagrams of the I-Ching as a pattern and form waveform packets that simulate the role of the mDNA (Messenger DNA) transmitting information to the ribosomes of the cells for the synthesis of essential amino acids according to a table of correspondence between the 64 hexagrams of the I-Ching and the 20 amino acids of the DNA.

## Description

### Technical field of Invention

This invention refers to a neurostimulation apparatus and method, and in particular to a neurostimulation apparatus and method that sends electrical signals to the ribosomes of the cells, simulating the role of the mDNA (Messenger DNA), to create amino acids and polypeptide chains.

The invention implements a portable apparatus composed of a set of 4 electrical therapy modules (HL, FL, HR and FR) configured to operate in a local communication network, for example Bluetooth Low Energy (BLE), maintain an external communication by Bluetooth Low energy (BLE), generate two-phase direct micro-current signals, with a selectable exposure time, level and frequency and multiplexed output, and encoded waveform packets according to a method that relates, by means of a table of Correspondence, the 6 lines of the 64 hexagrams of the I-Ching with the decoding of the DNA in its 20 amino acids, to generate and transmit neurostimulation to living beings that simulates the role of the mDNA (Messenger DNA) transmitting information to the ribosomes of the cells for the synthesis of essential amino acids and polypeptide chains.

### Background of the Invention

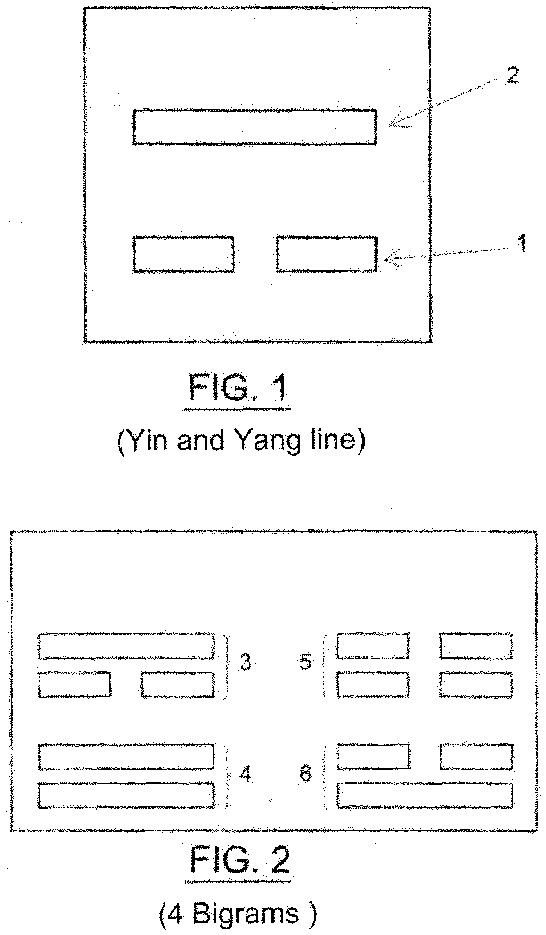

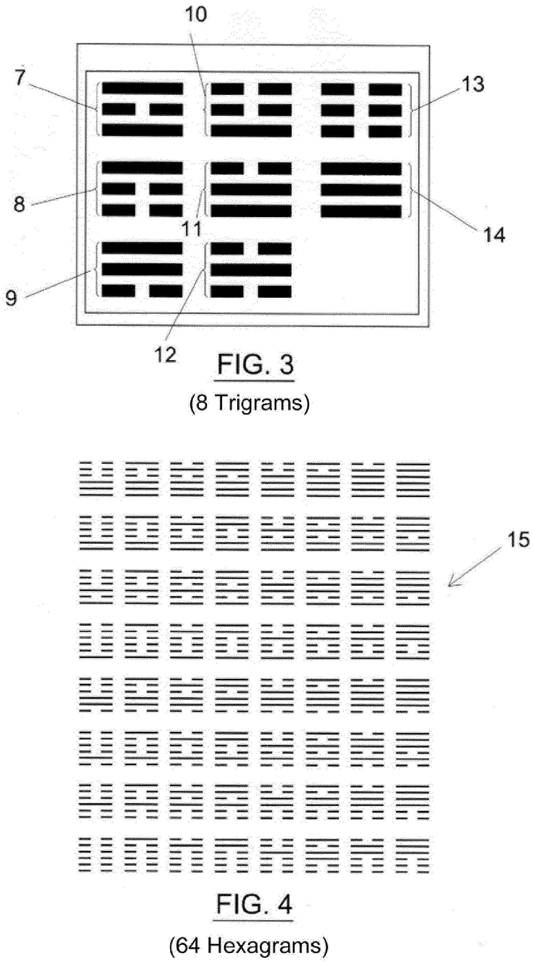

The I-Ching is a classic text of Chinese philosophy that was developed by the legendary Fu Hsi about 5000 years ago in prehistoric China. The main idea exposed in the I-Ching is the concept of change, that reality is in constant movement. It is a system of linear symbols that compose 64 different configurations of 6 lines called hexagrams, which represent different vital situations. Each line can be Yin (represented by a broken line marked as reference 1 in Figure 1) or Yang (continuous line marked as reference 2 in said Figure 1). In addition, each Yin or Yang line can be stable or mobile, which gives rise to the 4 types of possible lines called bigrams and that are the following: Old Yin, Young Yin, Young Yang and Old Yang (indicated respectively with the numerical references 4, 3; 6, 5 in Fig. 2). The combination of three Yin or Yang lines results in 8 different trigrams, which represent 8 basic attitudes to work with Yin and Yang energies (see the groupings of lines indicated with the numerical references 7 to 12 shown in the representations of Fig. 3). Finally, the combination of six lines or two trigrams gives rise to each of the 64 possible hexagrams that are jointly marked with the numerical reference 15 and can be seen in Fig. 4 of the drawings. Each hexagram has a name and related text, and represents a certain situation. At the internal level, I-Ching represents the basis of the energetic work that nourishes Taoist meditation. Meditation is at the same time the basis of many disciplines of Chinese origin, for example, Chi Kung, Tai chi, Feng Shui, massage, acupuncture ... etc. In addition, modern scientists who have studied the philosophy of I-Ching have discovered fascinating parallelisms between I-Ching and mathematics, modern physics and genetics, suggesting that I-Ching is universal in nature.

In 1953, scientists James Watson and Francis Crick determined the structure of deoxyribonucleic acid (DNA) and in the early 60s, the way in which DNA stores information was decoded giving rise to the genetic code.

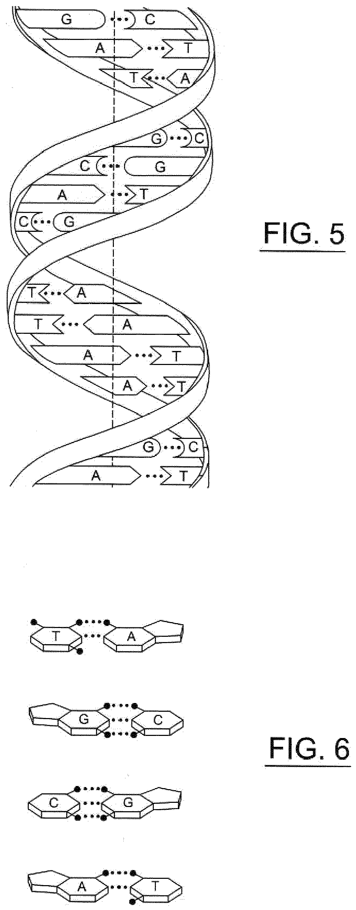

It is known that DNA is the hereditary material present in all living things that is responsible for transmitting genetic information between the progenitors and their offspring. DNA is located in the nucleus of the cells and consists of two strands composed of multiple chained units, known as nucleotides, which are entwined forming a double helix (see fig. 5). These nucleotides are composed in turn of three chemical compounds: a pentose (sugar) called deoxyribose, a phosphate group and a nitrogenous base. The first two components remain fixed forming the DNA skeleton while the nitrogenous bases are variable and they are responsible for storing the DNA information. There are two types of nitrogenous bases: purine and pyrimidine, which in turn are divided into two subtypes in the DNA: adenine (A) and guanine (G) are purine bases, and cytosine (C) and thymine (T) are pyrimidine bases. The two strands of DNA are complementary, as (A) of one of the strands always links to (T) of the opposite strand, and (G) links to (C). Each pair (A-T) or (G-C) is called "Base pair", and coupling between (A) and (T) takes place by means of two chemical bonds, known as "double hydrogen bond", and between (G) and (C) by means of three bonds known as "triple hydrogen bond", as represented in Fig. 6. Thus, the letters (A, G, C and T) represent the alphabet which forms the DNA language.

Amino acids are chemical compounds formed by an amino group and a carboxyl group. When two amino acids are combined a dipeptide is obtained; three amino acids give a tripeptide and a greater number of amino acids form polypeptides. When the polypeptide consists of more than fifty amino acids it is called protein.

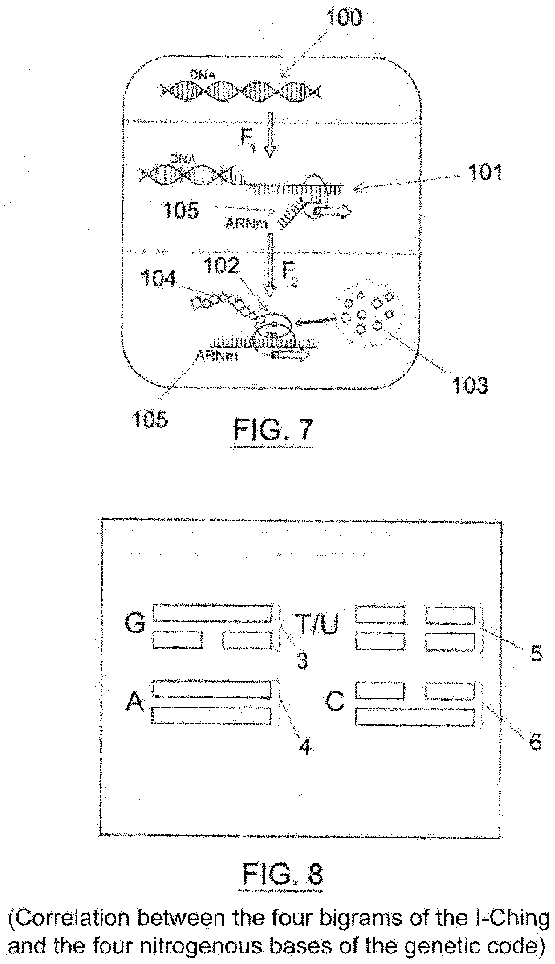

The synthesis of proteins occurs in two stages. In the first stage, graphically indicated in Figure 7 by arrow F₁, called transcription, the permanent message of DNA 100 is copied to a temporal messenger RNA, shown graphically in Figure 7 with numerical reference 105, by an enzyme (RNA Polymerase 101). This message in the form of mRNA can be read by a complex cellular "machinery" called ribosome 102. In this second stage, called "translation" and indicated by arrow F₂, the ribosome 102 assembles amino acids 103 in the order specified in mRNA 105 to create the specific protein 104.

A number of studies have revealed impressive relationships between the structure and dynamics of I-Ching and DNA. The main studies carried out so far include those by the German biologist Gunther S. Stent (1969), the German biochemist Martin Schönberger (1973), Dr. Frank Fiedeler (1977), the physiologist Johnson FaaYan (1991), the American mathematician Katya Walter (1996), and a variety of documents that are available on the Internet.

### Explanation of the invention

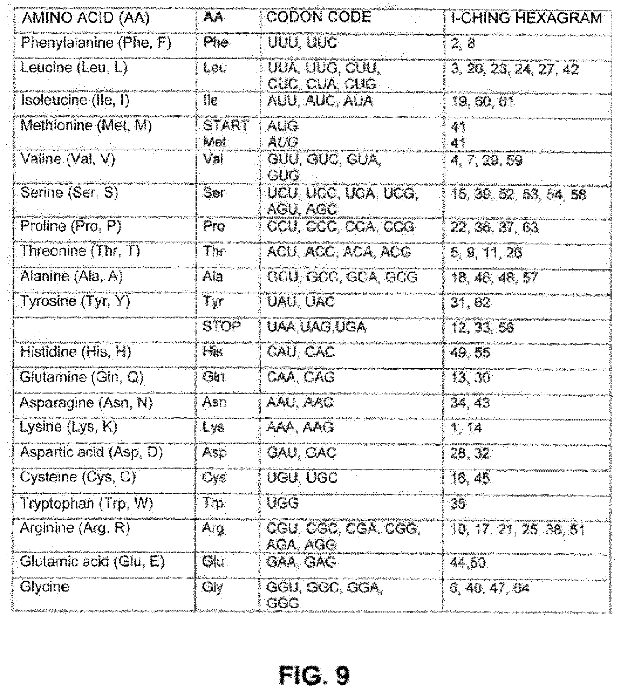

The Neurostimulation method that simulates the role of mDNA (Messenger DNA) for the formation of essential amino acids and polypeptide chains in this invention is based on the correlation proposed by the German biochemist Martin Schönberger (1973) between the four bigrams of I-Ching (Figure 2) and the four nitrogenous bases of the genetic code (Figure 5) as follows: Guanine (G) = Young Yang 3, Cytosine (C) = Young Yin 6, Adenine (A) = Old Yang 4 and Thymine (T)/Uracilo (U) = Old Yin 5. This correlation has been outlined in Figure 8 of the drawings, whereby the 20 essential amino acids of the DNA are related with the 64 hexagrams (Figure 4) of the I-Ching according to the table shown in Figure 9 of the drawings.

This invention refers to a method and apparatus that sends electrical signals to the ribosomes of the cells, simulating the role of the mDNA (Messenger DNA), to create amino acids and polypeptide chains.

### Brief description of the drawings

The above and other features and advantages will be more fully understood from the following detailed description of some examples of embodiment with reference to the accompanying drawings, in which:
Fig. 1 shows the representation of the Yin and Yang lines;
Fig. 2 shows the four bigrams of the I-Ching;
Fig. 3 shows the representation of the 8 trigrams of the I-Ching;
Fig. 4 shows the representation of the 64 hexagrams of the I-Ching;
Fig. 5 shows a representation of DNA
Fig. 6 shows the chemical bonds between the nitrogenous bases;
Fig. 7 shows transcription and translation processes in protein formation;
Fig. 8 shows the correlation between the four bigrams of the I-Ching and the four nitrogen bases of the genetic code;
Fig. 9 shows the table of correspondence of the I-Ching with the decoding of DNA into its 20 amino acids;
Fig. 10 shows the encoding of Yin and Yang lines with pulse modulation;
Fig. 11 shows the example of a hexagram with pulse modulation;
Fig. 12 shows an example of 4 amino acids hexagrams electric pulses;
Fig. 13 shows the configuration of the local and external Bluetooth Low Energy (BLE) networks between the independent modules HL, FL, HR and FR;
Fig. 14 shows the connection of the four modules HL, FL, HR and FR in the body, and

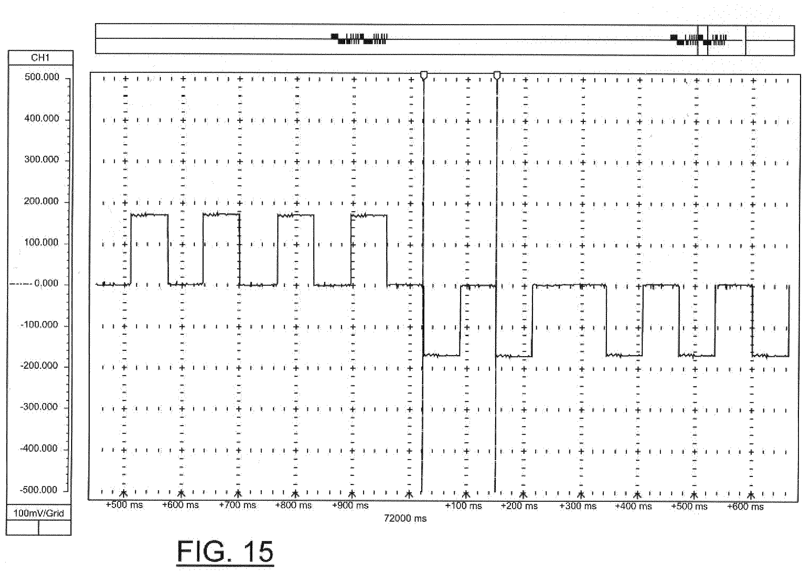

Fig. 15 shows an example of an output signal;

### Description of the Invention

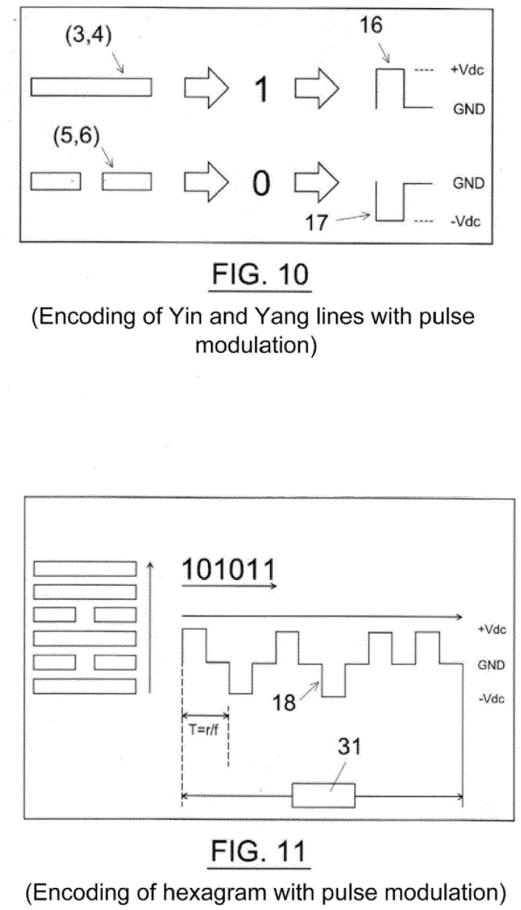

The Neurostimulation method that simulates the role of mDNA (Messenger DNA) for the formation of essential amino acids and polypeptide chains uses very low-intensity (micro-current) two-phase electrical signals (see Figure 10 of the drawings) that relate the Yang lines 3, 4 of the I-Ching with positive pulses 16, with a 50% cycle ratio and the Yin lines 5, 6 with negative pulses 17, with a 50% cycle ratio, and with a pulse sequence according to the decoding between amino acid/ I-Ching Hexagram shown in table of Fig. 9.

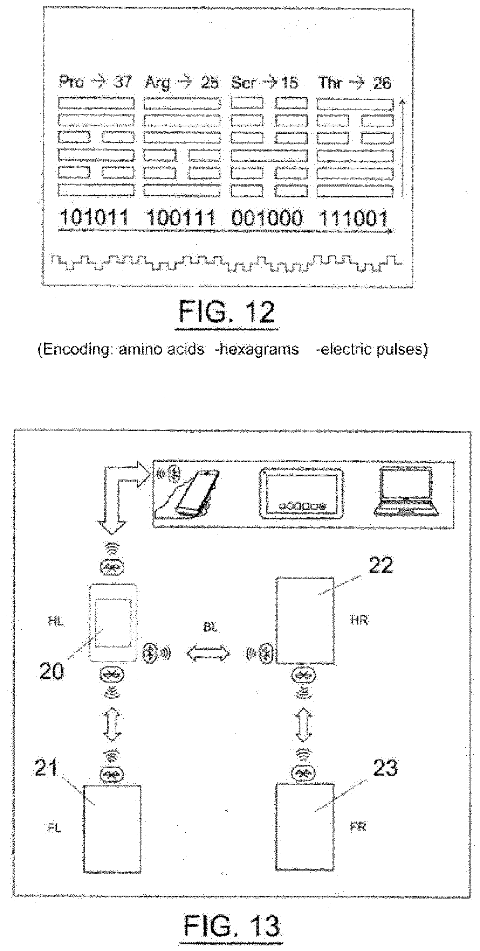

First it is decided based on the amino acid needed, then the hexagram is found in group 15 of Figure 4 that represents it (there may be redundancy of hexagrams, namely, several hexagrams for the same amino acid) and the lines of the hexagram translate into pulses following an order from bottom to top and left to right as shown in graph 18 of positive and negative values shown in Fig. 11. Several consecutive amino acids form a polypeptide chain and an example of mDNA simulation by electrical signals can be seen in Fig. 12. Normally, the packages of polypeptide chains begin with an amino acid which marks the start of the chain (START = (AUG) and end with one of the three amino acids which mark the end of the chain (STOP = UAA, UAG and UGA) to simulate the mRNA of the polypeptide chain that is the messenger that carries the information to the cell ribosomes to create the polypeptides.

The electrical signals are applied to the skin at sites marked by Traditional Chinese Medicine (TCM) which are chosen according to the pathologies, diagnosis and treatment techniques, and are characterized by 4 levels of direct current with a very low intensity (between 50µA and 200µA), frequency and duration of treatment. These characteristics and others that facilitate the application of this kind of neurostimulation are selected on the apparatus described below.

The invention implements a portable apparatus composed of a set of 4 electrical therapy modules (HL, FL, HR and FR) configured to operate in a local communication network, for example Bluetooth Low Energy (BLE), maintain an external communication by Bluetooth Low energy (BLE), generate two-phase direct micro-current signals, with a selectable exposure time, level and frequency and multiplexed output, and encoded waveform packets according to a method that relates, by means of a table of Correspondence, the 6 lines of the 64 hexagrams of the I-Ching with the decoding of the DNA in its 20 amino acids, to generate and transmit neurostimulation to living beings that simulates the role of the mDNA (Messenger DNA) transmitting information to the ribosomes of the cells for the synthesis of essential amino acids and polypeptide chains.

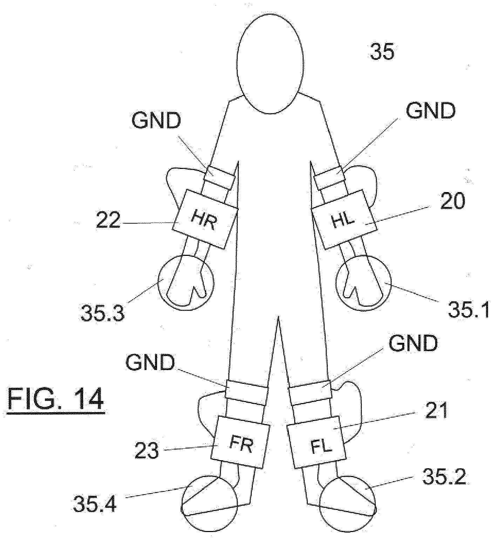

The portable electronic apparatus according to the invention comprises:
- a set of 4 small modules referred to above as HL, FL, HR and FR, which in Figure 13 have been identified using the numerical references 20, 21, 22, 23, respectively, these being light and independent, automatically connected to each other by a wireless local communications network via radio, such as Bluetooth Low Energy 24, 25, and connected by another low energy Bluetooth wireless line 26 with external units forming part of a Global System (Fig. 13); and,
- Each independent module 20, 21, 22, 23 (Fig. 13) is powered (POWER) by a 28.1, 28.2, 28.3, 28.4 rechargeable 3 .7V lithium battery, and contains a power supply to recharge the battery from an external micro USB connector (not shown in the figure), raise the voltage to +5V and generate another auxiliary voltage of -5V; and,
- Each independent module 20, 21, 22, 23 (Fig. 13) contains a microprocessor (µP) referenced as 29.1, 29.2, 29.3, 29.4, respectively, with a software that manages communications 24, 25, activation of relative signal generators 30, and distribution to the respective output connector 31; and,
- Each independent module 20, 21, 22, 23 (Fig. 13) contains radio communications transmitters/receivers, as in this case Bluetooth Low Energy 24, 25, 26 so that each independent module 20, 21, 22, 23 (Fig. 13) contains a signal generator 30, which in the example embodiment, is responsible for generating two-phase direct micro-current signals, selecting the level and frequency, and multiplexing the output (OUT); and,
- Each independent module 20, 21, 22, 23 (Fig. 13) controls in stand-alone and/or synchronized mode with the rest of the modules the intensity and frequency, waveform and signal output; and,
- Each module 20, 21, 22, 23 is mounted on a flexible strap 32 (see Fig. (14) which adjusts to a user's wrists and ankles; In the inner part of the strap and in contact with the skin there is a strip of metal fabric that ensures the ground contact between each module and the skin by means of a small cable 33 that connects the strap with the module as shown in Fig. 14; and,
- Each module 20, 21, 22, 23 has a small wire harness 34, visible in Figure 14, which connects the signal output with the dots on the skin where they need to be applied; and,
- a specific firmware of each microprocessor 29.1 - 29.4 manages the functions of each module, while another microprocessor software of the touch screen 27 manages the Man/Machine interface (MMI) to select the characteristics of the waveforms to send and other ancillary functions.

The apparatus is capable of selecting, during the session, the reproduction of short-duration sounds and images or videos that help user treatment,

The apparatus can be used using the 4 modules at the same time or using the following partial configurations: (HL, FL, HR, FR), (HL, FL), (HL, FL, HR), (HL, HR) and (HL, HR, FR).

### Industrial Applicability

The invention is applicable within the medical sector, for the purpose of neurostimulation to simulate the role of mDNA (Messenger DNA) in order to create essential amino acids and polypeptide chains.

## Claims

1. Neurostimulation method, **characterized in that** the neurostimulation is carried out by means of two-phase direct micro-current electrical signals, and encoded waveform packets (51) that relate by means of a table of correspondence the 6 lines of the 64 hexagrams (15) of the I-Ching with the decoding of the DNA in its 20 amino acids, to generate and transmit electrical signals to the ribosomes of the cells of living beings, simulating the role of the mDNA (Messenger DNA), to create essential amino acids and polypeptide chains

2. Neurostimulation apparatus, in particular a portable electronic apparatus for the implementation of the neurostimulation method of claim 1, **characterized in that** it consists of: a set of 4 small light and Independent modules (20, 21, 22, 23) automatically connected to each other by a local wireless low-energy radio communication network (Bluetooth), and connected by another low-energy radio (Bluetooth) wireless line (26) with external units to form part of a Global system.
